(19) **Europäisches Patentamt**

**European Patent Office**

**Office européen des brevets**

(11) Veröffentlichungsnummer : **0 397 646 B1**

(12) **EUROPÄISCHE PATENTSCHRIFT**

(45) Veröffentlichungstag der Patentschrift :
**08.07.92 Patentblatt 92/28**

(51) Int. Cl.⁵ : **A61K 33/04, A61K 33/30,**
**// (A61K33/04, 31:315,**
**31:195), (A61K33/30, 33:04,**
**31:195)**

(21) Anmeldenummer : **90890146.5**

(22) Anmeldetag : **11.05.90**

(54) Pharmazeutisches Präparat zur Behandlung von Lebererkrankungen.

(30) Priorität : **11.05.89 AT 1130/89**

(43) Veröffentlichungstag der Anmeldung :
**14.11.90 Patentblatt 90/46**

(45) Bekanntmachung des Hinweises auf die
Patenterteilung :
**08.07.92 Patentblatt 92/28**

(84) Benannte Vertragsstaaten :
**AT BE DE DK ES FR GB GR IT LU NL SE**

(56) Entgegenhaltungen :
**EP-A- 0 048 473**
**DE-A- 1 792 034**

(56) Entgegenhaltungen :
**CHEMICAL ABSTRACTS, Band 93, Nr. 9, 1.**
**September 1980, Zusammenfassung Nr.**
**89616w, Columbus, Ohio, US; A.J. BLOTCKY et**
**al.:"Organ content of selenium, zinc, magne-**
**sium, calcium and copper in alcoholic cirrhotic**
**patients and controls", & TRACE SUBST.EN-**
**VIRON. HEALTH 1979, 13, 417-24**

(73) Patentinhaber : **HOMOSAN AG**
**Seestrasse 79**
**CH-8806 Bäch (CH)**

(72) Erfinder : **Mittheiss, Elisabeth, Mag.**
**Gardegasse 3/5**
**A-1070 Wien (AT)**
Erfinder : **Gusenbauer, Werner, Mag.**
**A-3623 Kottes (AT)**

(74) Vertreter : **Müllner, Erwin, Dr. et al**
**Patentanwälte Dr. Erwin Müllner Dipl.-Ing.**
**Werner Katschinka Weihburggasse 9/24**
**A-1010 Wien (AT)**

**Beschreibung**

Die vorliegende Erfindung betrifft ein pharmazeutisches Präparat zur Behandlung von Lebererkrankungen. Das Leberschutzpräparat kann zur Behandlung von metabolischen und toxischen Lebererkrankungen in der Human- und Veterinärmedizin eingesetzt werden. Insbesondere kann es bei alkoholischer und toxischer Lebererkrankung, Hyperammonämie, Abstinenzerscheinungen und Alkoholdelirium sowie akuter und chronischer Cerebralhypoxie verwendet werden.

Andauernder Alkoholkonsum und/oder Streß kann eine Vielfalt von Lebererkrankungen hervorrufen. Die zytologischen Veränderungen reichen von einfacher Fettleber über verschiedene Stufen der Alkoholhepatitis und der Fibrose bis zur Zirrhose.

Im Verlauf des Alkoholabbaues ist das NAD (Nicotinamid-adenin-dinucleotid) Protonenakzeptor und wird zu NADH reduziert. Durch das Ansteigen der Menge NADH wird das NADH/NAD-Verhältnis erhöht, wodurch der Citronensäurecyclus (Krebs-Cyclus) gehemmt wird: wegen des Mangels an NAD ist die Gluconeogenese aus Aminsäuren gehemmt. Der Alkohol wird durch folgende Enzyme abgebaut: Alkoholdehydrogenase, MFOS (mischfunktionelles Oxydationssystem), Katalase, NADP (Nicotin-adenin-dinucleotidphosphat)-Oxydase, Xanthinoxydase. Beim Abbau des Alkohols mittels Xanthinoxidase und Katalase entsteht Wasserstoffperoxyd und daraus das freie Sauerstoffradikal. Acetaldehyd ist der Endstoff aller dieser metabolischen Prozesse. Beim Abbau des Acetaldehyds ist das Sauerstoffradikal der Hauptmetabolit, welches die Lipidoxydation verursacht.

Bei langanhaltender Alkoholexposition und der dadurch bedingten Leberschädigung muß man in erster Linie mit zwei toxischen Produkten rechnen: freie Sauerstoffradikale und Acetaldehyd. Bei der Abwehr ihrer toxischen Wirkungen spielen die reduzierte Form des Glutathions und andere, die Thiolgruppe enthaltende Aminosäuren, eine sehr große Rolle. Hepatotoxizität und Abfall der Glutathionkonzentration sind direkt proportional.

Ziel der Erfindung ist die Bereitstellung eines Präparates mit dem es möglich ist, die insbesondere durch Alkohol aber auch durch Streß hervorgerufenen Schädigungen der Leber zu beseitigen oder zumindest zu mildern.

Dieses Ziel wird mit einem Präparat erreicht, da gegebenenfalls neben herkömmlichen Arzneimittelträgern, Bindemitteln und/oder Hilfsstoffen als wirksamen Bestandteil eine Mischung aus

a) L-α-Amino-bernsteinsäure (L-Asparaginsäure)
b) L-β-Merkapto-alanin (L-Cystein)
c) L-α-Amino-glutarsäure (L-Glutaminsäure)
d) Natriumselenat ($Na_2SeO_4$) und
e) Zinkacetat ($Zn(CH_3OO)_2$) oder Zinksulfat ($ZnSO_4$) enthält.

Erfindungsgemäß enthält das pharmazeutische Präparat die Wirksubstanzen a bis e im Gewichtsverhältnis (0,020-2,00): (0,025-1,00) : (0,025-5,00) : (28.10$^{-6}$ -500.10$^{-6}$) : (0,010-0,300).

Das neue Leberschutzpräparat hilft bei therapeutischer Anwendung die metabolischen und toxischen Wirkungen des Alkohols sowie von Streßbeanspruchungen zu vermeiden. Das zur Sicherung des Energiebedarfs des Stoffwechselprozesses nötige ATP-Niveau wird erhöht, und die toxischen, freien Radikale werden gebunden.

Die Glutaminsäure und das Cystein spielen eine wichtige Rolle bei der Bildung des Glutathions, welches bei den Entgiftungsprozessen der Leber wichtig ist, und dessen Konzentration in den Leberzellen unter der Wirkung von Xenobiotika, oxidativ schädigenden Substanzen und Alkohol sinkt, wodurch sein hepatozellulärer Schutz vermindert wird.

Selen ist eine strukturelle Komponente der Glutathionperoxidase. Dieses Enzym katalysiert die Reduktion des $H_2O_2$ und schützt die Zellen vor oxidativen Schäden. Deshalb ist bei oxidativen Streßzuständen die Selen enthaltende Glutathionperoxydase die Schlüsselsubstanz des antioxidativen Systems. Selen bewirkt eine Erhöhung der Glutathionkonzentration. Selen verhindert somit Lebeschädigungen, welche durch hepatotoxische Stoffe hervorgerufen wurden.

Histologische Untersuchungen haben die Verminderung der Kollagenakkumulation bei Präventivbehandlung mit Zink bestätigt. Zink ist ein wirksames antifibrotisch wirkendes Element. Asparaginsäure, Glutaminsäure und Cystein dienen der Synthese von ATP (Adenosin-5′-triphosphat). Durch enzymatische Prozesse im Zytoplasma und in den Mitochondrien der Leberzelle entsteht aus Asparaginsäure Oxalessigsäure und aus Glutaminsäure α-Ketoglutarsäure. Die α-Ketoglutarsäure kann über Oxalessigsäure auch in Brenztraubensäure, bzw. über einen anderen Reaktionsweg in Succinyl-Co A übergehen und auf diese Weise an der Gluconeogenese und an der Energieproduktion teilnehmen.

Ketosäuren sind direkte Reaktionsprodukte des Citronensäurecyclus, wobei die Oxalessigsäure die Schlüsselverbindung der Gluconeogenese ist. Der Transport des zur ATP-Synthese benötigten anorganischen Phosphors erfolgt mit Hilfe eines durch die Thiolgruppe des L-Cysteins aktivierten Trägers.

Die gleichzeitige Anwendung der drei Aminosäuren hemmt den durch die Blockierung der Gluconeogenese entstandenen Katabolismus der Eiweiße und Fettsäuren, wodurch die Anreicherung und Konzentrationssteigerung von Ketonsäuren, Glycerin und Triglyceriden vermindert wird. Gleichzeitig verbessert sich die Aktivität des Ionentransportes, die Intensität der biosynthetischen Prozesse und die osmotischen Funktionen. Die Konzentrationserhöhung der Glutamin- und Asparaginsäuremetaboliten vermindert die Intensität der Glycolyse im zytoplasmatischen Bereich. Aufgrund des funktionellen Zusammenhanges von Citronensäure- und Harnstoffzyklus verbessert sich der Mechanismus der Ammoniakeliminierung parallel mit der mitochondrialen oxidativen Phosphorylierung.

Das Leberschutzpräparat hat den Vorteil, daß es aus Naturstoffen besteht und praktisch keine Toxizität aufweist, ausgenommen in höchster Dosierung bei niereninsuffizienten Patienten.

Zusammenfassend kann gesagt werden, daß die drei Aminosäuren, L-Asparagin-, L-Glutaminsäure und L-Cystein eine Erhöhung des ATP-Niveaus begünstigen, welches für den Ablauf der biosynthetischen Prozesse notwendig ist. Sie spielen eine wichtige Rolle bei der Bindung von toxischen freien Radikalen, der Beschleunigung der Ammoniakeliminierung und bei der Produktion des Glutathions in der Leber. Gemeinsam mit den Elementen Selen und Zink stellen sie eine neue leberwirksame Arzneimittelspezialität dar.

Das Leberschutzpräparat wird vorzugsweise oral in Form von Kapseln und parenteral in Form von Infusionen verabreicht. Bei oraler Verabreichung beträgt die durchschnittliche Tagesdosis 1,00 g L-Asparaginsäure, 0,50 g L-Cystein, 2,00 g L-Glutaminsäure, 200,0 µg Natriumselenat und 0,150 g Zinkacetat. Bei parenteraler Verabreichung in Form von Infusionen beträgt die Tagesdosis 2,00 - 6,00 g L-Asparaginsäure, 0,50 - 2,00 g L-Cystein, 5,00 - 20,00 g L-Glutaminsäure, 50,00 - 500,00 µg Natriumselenat und 0,10 - 0,30 g Zinkacetat.

Die Behandlungsart und die Tagesdosis ist abhängig vom Schweregrad der Lebererkrankung. Patienten mit ausgeprägter Alkoholhepatitis und Zeichen einer beginnenden Leberinsuffizienz erhalten in der ersten Zeit Infusionslösungen, dann wird die Behandlung über mehrere Monate mit Kapseln weitergeführt. Veterinärdosierungen werden in Abhängigkeit vom Körpergewicht verabreicht. Die durchschnittliche Tagesdosis pro kg Körpergewicht ist 0,015 g L-Asparaginsäure, 0,0075 g L-Cystein, 0,03 g L-Glutaminsäure, 3,00 µg Natriumselenat und 0,002 g Zinkacetat.

BEISPIEL 1:

Eine Kapsel kann wie folgt hergestellt werden:

Inhaltsstoffe

| | |
|---|---|
| L-Asparaginsäure | 0,150 g |
| L-Cystein | 0,100 g |
| L-Glutaminsäure | 0,250 g |
| Na-Selenat | 100 µg |
| Zinkacetat | 0,050 g |

Die Inhaltesstoffe werden gut gemischt und die Mischung wird in Kapseln abgefüllt.

BEISPIEL 2:

Eine Infusionslösung kann pro 1000 ml eine Wirkstoffmischung folgender Zusammensetzung enthalten:

| | |
|---|---|
| L-Asparaginsäure | 4,0 g |
| L-Cystein | 1,0 g |
| L-Glutaminsäure | 9,0 g |
| Na-Selenat | 200 µg |
| Zinkacetat | 0,100 g |

BEISPIEL 3:

Eine Infusionslösung kann pro 1000 ml eine Wirkstoffmischung folgender Zusamensetzung enthalten:

| | |
|---|---|
| L-Asparaginsäure | 4,00 g |
| L-Cystein | 2,00 g |
| L-Glutaminsäure | 10,00 g |

EP 0 397 646 B1

| Na-Selenat | 200,00 µg |
|---|---|
| Zinkacetat | 0,10 g |

## Patentansprüche

1. Pharmazeutisches Präparat zur Behandlung von Lebererkrankungen, dadurch gekennzeichnet, daß es als wirksamen Bestandteil eine Mischung aus

a) L-$\alpha$-Amino-bernsteinsäure (L-Asparaginsäure)

b) L-$\beta$-Merkapto-alanin (L-Cystein)

c) L-$\alpha$-Amin-glutarsäure (L-Glutaminsäure)

d) Natriumselenat ($Na_2SeO_4$) und

e) Zinkacetat ($Zn(CH_3OO)_2$) oder Zinksulfat ($ZnSO_4$) enthält.

2. Pharmazeutisches Präparat nach Anspruch 1, dadurch gekennzeichnet, daß es die Wirksubstanzen a bis e im Gewichtsverhältnis (0,020-2,00) : (0,025-1,00) : (0,025-5,00) : ($28.10^{-6}$ -$500.10^{-6}$) : (0,010-0,300) enthält.

3. Pharmazeutisches Präparat nach den Ansprüchen 1 und 2, dadurch gekennzeichnet, daß es in Kapseln vorliegt, welche je 0,150 g L-Asparaginsäure, 0,100 g L-Cystein, 0,250 g L-Glutaminsäure, 100 µg Natriumselenat und 0,050 g Zinkacetat enthalten.

4. Pharmazeutisches Präparat nach den Ansprüchen 1 und 2, dadurch gekennzeichnet, daß es als Infusionslösung vorliegt, die pro 1000 ml 4,0 g L-Asparaginsäure, 1,0 g L-Cystein, 9,0 g L-Glutaminsäure, 200 µg Natriumselenat und 0,100 g Zinkacetat enthält.

5. Pharmazeutisches Präparat nach der Anspruch en 1 und 2, dadurch gekennzeichnet, daß es als Infusionslösung vorliegt, die pro 1000 ml 4,00 g L-Asparaginsäure, 2,00 g L-Cystein, 10,00 g L-Glutaminsäure, 200,00 µg Natriumselenat und 0,10 g Zinkacetat enthält.

## Claims

1. A pharmaceutical preparation for the treatment of diseases of the liver, characterised in that bit contains as effective component a mixture of:

a) L-$\alpha$-amino-succinic acid (L-aspartic acid)

b) L-$\beta$-mercapto-alanine (L-cysteine)

c) L-$\alpha$-amine-glutaric acid (L-glutamic acid)

d) sodium selenate ($Na_2SeO_4$ ) and

e) zinc acetate ($Zn(CH_3OO)_2$) or zinc sulphate ($ZnSO_4$).

2. A pharmaceutical preparation according to Claim 1, characterised in that it contains the effective substances a to e in the weight ratio (0.020-2.00) : (0.025-1.00) : (0.025-5.00) : ($28.10^{-6}$ -$500.10^{-6}$ ) : (0.010-0.300).

3. A pharmaceutical preparation according to Claims 1 and 2, characterised in that it is present in capsules which each contain 0.150 g L-aspartic acid, 0.100 g L-cysteine, 0.250 g L-glutamic acid, 100 µg sodium selenate and 0.050 g zinc acetate.

4. A pharmaceutical preparation according to Claims 1 and 2, characterised in that it is present as an infusion solution, which contains per 1000 ml 4.0 g L-aspartic acid, 1.0 g L-cysteine, 9.0 g L-glutamic acid, 200 µg sodium selenate and 0.100 g zinc acetate.

5. A pharmaceutical preparation according to Claims 1 and 2, characterised in that it is present as an infusion solution, which contains per 1000 ml 4.00 g L-aspartic acid, 2.00 g L-cysteine, 10.00 g L-glutamic acid, 200.00 µg sodium selenate and 0.10 g zinc acetate.

## Revendications

1. Préparation pharmaceutique pour le traitement de maladies du foie caractérisée en ce qu'elle contient comme constituant actif un mélange de:

a) Acide L-$\alpha$-aminosuccinique (L-asparagine)

b) Acide L-$\beta$-mercapto-alanine (L-cystéine)

c) Acide L-$\alpha$-aminoglutarique (L-glutamine)

d) Sélénate de sodium ($Na_2SeO_4$) et

e) Acétate de zinc ($Zn(CH_3OO)_2$ ou sulfate de zinc ($ZnSO_4$)

2. Préparation selon la revendication 1, caractérisée en ce qu'elle contient les substances actives de a) à e) dans le rapport pondéral (0,020-2,00) : (0,025-1,00) : (0,025-5,00) : ($28.10^{-6}$-$500.10^{-6}$) : (0.010-0,300).

3. Préparation pharmaceutique selon les revendications 1 et 2, caractérisée en ce qu'elle se trouve dans des capsules qui contiennent chacune 0,150 g de L-asparagine, 0,100 g de L-cystéine, 0,250 g de L-glutamine, 100 µg de sélénate de sodium et 0,050 g d'acétate de zinc.

4. Préparation pharmaceutique selon les revendications 1 et 2, caractérisée en ce qu'elle se trouve sous forme de solution pour infusion qui contient pour 1000 ml, 4,0 g de L-asparagine, 1,0 g de L-cystéine, 1,0 g de L-glutamine, 200 µg de sélénate de sodium et 0,100 g d'acétate de zinc.

5. Préparation pharmaceutique selon les revendications 1 et 2, caractérisée en ce qu'elle se trouve sous forme de solution pour infusion qui contient pour 1000 ml, 4,0 g de L-asparagine, 2,0 g de L-cystéine, 10,00 g de L-glutamine, 200 µg de sélénate de sodium et 0,10 g d'acétate de zinc.